# EUROPÄISCHE PATENTSCHRIFT

(11) **EP 1 648 852 B1**
(45) Veröffentlichungstag und Bekanntmachung des Hinweises auf die Patenterteilung: **17.06.2009**
(21) Anmeldenummer: 04740986.7
(22) Anmeldetag: 14.07.2004
(51) Int. Cl.: C07C 51/58, C07C 63/10

(54) **VERFAHREN ZUR HERSTELLUNG VON SUBSTITUIERTEN ARYLCARBONS URE CHLORIDEN**
METHOD FOR PRODUCING SUBSTITUTED ARYLCARBOXYLIC ACID CHLORIDES
PROCEDE DE PRODUCTION DE CHLORURES-ACIDES ARYLCARBOXYLIQUES SUBSTITUES

(30) Priorität: 17.07.2003 DE 10332485
(43) Veröffentlichungstag der Anmeldung: 26.04.2006
(73) Patentinhaber: BASF SE, 67056 Ludwigshafen (DE)
(72) Erfinder: MAASE, Matthias, 67346 Speyer (DE); BERTLEIN, Gerhard, 69151 Neckargemünd (DE); GANZ, Holger, 67067 Ludwigshafen (DE); DUST, Matthias, 67071 Ludwigshafen (DE)
(74) Vertreter: Isenbruck, Günter
(86) Internationale Anmeldenummer: PCT/EP2004/007768
(87) Internationale Veröffentlichungsnummer: WO 2005/012219

(56) Entgegenhaltungen:
- EP-A- 0 009 205
- EP-A- 0 706 987
- US-A- 3 187 057
- US-A- 3 691 217

## Beschreibung

Die Erfindung betrifft ein Verfahren zur Herstellung von substituierten Arylcarbonsäurechloriden.

2,4,6-Trimethylbenzoylchlorid (TMBC) ist ein wichtiger Rohstoff zur Herstellung von Photoinitiatoren des Acylphosphinoxidtyps, beispielsweise von TPO (Trimethylbenzoyldiphenylphosphinoxid).

TMBC kann, wie in EP-A 0 554 679 beschrieben, in einer vierstufigen Synthese hergestellt werden. Dabei wird in einer ersten Stufe Mesitylen mit Chloressigsäurechlorid zum Chlortrimethylacetophenon umgesetzt. In einer zweiten Stufe wird aus Chloracetophenon durch Umsetzung mit Natriumhypochlorit Trichlortrimethylacetophenon erhalten. In einer dritten Stufe wird Trichloracetophenon mit Natronlauge zum Trimethylbenzoesäure-Natriumsalz umgesetzt und aus diesem durch Ansäuern mit Salzsäure Trimethylbenzoesäure erhalten. Schließlich wird in einer vierten Stufe durch Umsetzung mit Thionylchlorid aus der Trimethylbenzoesäure das Trimethylbenzoesäurechlorid erhalten.

Die Synthese ist aufgrund der Vielzahl von Synthesestufen aufwändig und durch schlechte Ausbeuten gekennzeichnet. Insbesondere muss das Zwischenprodukt Trimethylbenzoesäure vor der Umsetzung mit Thionylchlorid als Feststoff isoliert und getrocknet werden.

Analog lassen sich weitere ein- oder mehrfach alkylierte Benzoylchloride herstellen.

Eine weitere Methode zur Herstellung von TMBC wird in EP-A 0 706 987 beschrieben. Dabei wird Mesitylen in Anwesenheit von AlCl₃ zu Trimethylbenzoesäure carboxyliert und dieses anschließend mit Thionylchlorid zu TMBC chloriert. Auch diese Synthese ist durch schlechte Ausbeuten gekennzeichnet. So beträgt die Ausbeute des Carboxylierungsschritts nur 71%.

US 3,691,217 offenbart die Herstellung von Benzoylchlorid durch Umsetzung von Benzotrichlorid mit einer organischen Säure in Gegenwart von Zinntetrachlorid als Katalysator. In den Beispielen 1 bis 4 wird die Umsetzung von Benzotrichlorid mit verschiedenen aliphatischen und aromatischen Carbonsäuren (Laurinsäure, Methylbenzoesäure, Decansäure) in Gegenwart von SnCl₄ beschrieben.

US 3,187,057 offenbart ein Verfahren zur Herstellung von Trichlormethyl substituierten Polyalkylbenzolen durch Umsetzung von Trialkylbenzol oder Tetraalkylbenzol mit einem molaren Überschuss an CCl₄ in Gegenwart von Aluminiumchlorid. In Beispiel 1 wird wasserfreies Aluminiumchlorid in CCL₄ vorgelegt und bei 30°C Pentamethylbenzol in CCL₄ zugegeben. Anschließend wird der AlCl₃-Komplex durch Zugabe von 10%iger HCl-Lösung bei 10°C zerstört. Nach Phasentrennung und Entfernung von überschüssigem CCl₄ wird ein Roh-Trichlormethylpentamethylbenzol erhalten, welches anschließend zu Pentamethylbenzoesure hydrolisiert wird.

EP-A 0 009 205 offenbart ein Verfahren zur Herstellung von (substituiertem) Benzoylchlorid durch Umsetzungvon (substituiertem) Benzotrichlorid mit (substituierter) Benzoesäure und ggf. Wasser in Gegenwart eines Katalysators. Das Benzotrichlorid kann mit Halogen, Nitro oder Alkyl ein- oder zweifach substituiert sein. Als Katalysatoren werden verschiedene Mineralsäuren, Sulfonsäuren sowie M etallhalogenide genannt.

Aufgabe der Erfindung ist es, ein einfaches und wirtschaftliches Verfahren zur Herstellung von substituierten Benzoylchloriden bereitzustellen, das insbesondere durch verbesserte Ausbeuten gekennzeichnet ist. Aufgabe der Erfindung ist es insbesondere, ein einfaches und wirtschaftliches Verfahren zur Herstellung von TMBC bereitzustellen, das durch verbesserte Ausbeuten gekennzeichnet ist.

Gelöst wird die Aufgabe durch ein Verfahren zur Herstellung von mit C₁-C₄-Alkyl 3-, 4- oder 5-fach oder mit Halogen ein- oder mehrfach substituierten Benzoylchloriden (I), bei dem in einer ersten Stufe ein mit C₁-C₄-Alkyl 3-, 4- oder 5-fach oder mit Halogen ein- oder mehrfach substituiertes Benzol (II) mit CCl₄ in Gegenwart von AlCl3 und anschließender Hydrolyse des gebildeten AlCl3-Komplexes zum entsprechenden, mit C₁-C₄-Alkyl 3-, 4- oder 5-fach oder mit Halogen ein- oder mehrfach substituierten trichlormethylierten Aromaten (III) umgesetzt wird,
und in einer zweiten Stufe das trichlormethylierte substituierte Benzol (III) mit Wasser in Gegenwart eines Katalysators zum substituierten Benzoylchlorid (I) hydrolysiert wird, wobei in der zweiten Stufe die bei der Hydrolyse des AlCl₃-Komplexes anfallende wasserhaltige organische Phase eingesetzt und nach der Hydrolyse wasserfreies CCl₄ abdestilliert wird.

Durch das erfindungsgemäße Verfahren wird also die bekannte vierstufige Synthese durch eine zweistufige Synthese ersetzt, welche durch eine hohe Gesamtausbeute gekennzeichnet ist.

Ist das substituierte Benzol mit Halogen substituiert, so ist es bevorzugt mit Chlor substituiert.

Die erste Stufe des erfindungsgemäßen Verfahrens geht von einem substituierten Benzol (IIa) aus, das 3, 4 oder 5 C₁-C₄-Alkylreste (also Methyl, Ethyl, Prop-1-yl, Prop-2-yl, But-1-yl, But-2-yl, 2-Methylprop-1-yl und tert.-Butyl) aufweisen kann. Das substituierte Benzol kann auch Alkylsubstituenten und Halogensubstituenten (bevorzugt Chlor) nebeneinander aufweisen oder ausschließlich mit Halogen substituiert sein. Beispiele sind Chlorbenzol, Toluol, o-, m- und p-Xylol, Mesitylen, Pseudocumol, Hemellithol, Ethylbenzol und Cumol.

Insbesondere wird das erfindungsgemäße Verfahren eingesetzt, um aus Mesitylen (1,3,5-Trimethylbenzol) als substituiertem Benzol (II) 2,4,6-Trimethylbenzoylchlorid (Ib) herzustellen.

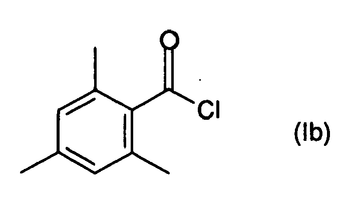

In der ersten Stufe wird das substituierte Benzol (II) mit CCl₄ in Gegenwart von AlCl₃ als Friedel-Crafts-Katalysator umgesetzt.

Das Molverhältnis CCl₄ zu Alkylaromat beträgt im Allgemeinen von 1 : 1 bis 15 : 1, bevorzugt von 1,5 : 1 bis 7 : 1.

Aus H. Hart und R. Fisch, J. Am. Chem. Soc. (1961), S. 4460-4466 und A. Siciliano, K. Nieforth, J. Med. Chem. (1971), S. 645-646 ist bekannt, die Friedel-Crafts-Alkylierung von Alkylaromaten mit CCl₄ mit einem Molverhältnis von CCl₄ zu Alkylaromat von 4 : 1 bis 13 : 1 durchzuführen. CCl₄ wird dabei nach der Reaktion vom Produkt abgetrennt und zweckmäßiger Weise zurückgeführt. Die Abtrennung großer CCl₄-Mengen ist aber aufwändig, weshalb es wünschenswert ist, mit einem möglichst geringen CCl₄-Überschuss zu arbeiten. Überraschender Weise wurde aber gefunden, dass die Friedel-Crafts-Reaktion mit guter Ausbeute bei einem geringen Molverhältnis von CCl₄ zu Alkylaromat von nur 1 : 1 bis 3,5 : 1, bevorzugt von 1,5 : 1 bis 2 : 1 verläuft. Daher wird gemäß einer Ausführungsform der Erfindung die Friedel-Crafts-Alkylierung des substituierten Benzols (II) mit einem Molverhältnis von CCl₄ zu Aromat von 1 : 1 bis 3,5 : 1, bevorzugt von 1,5 : 1 bis 2 : 1 durchgeführt.

Pro Äquivalent des substituierten Benzols (II), insbesondere Mesitylen, werden im Allgemeinen 1 bis 3, beispielsweise ca. 2 Äquivalente AlCl₃ eingesetzt. In einer Ausführungsform des erfindungsgemäßen Verfahrens beträgt dieses Verhältnis (Äquivalente AlCl₃ zu substituiertem Aromat) nur 1 bis 1,5, insbesondere 1 bis 1,3, speziell 1,1 bis 1,2. Es wurde gefunden, dass bei der Herstellung von TMBT aus Mesitylen die Menge an AlCl₃ bis auf einen sehr geringen Überschuss gesenkt werden kann, ohne dass die Ausbeute an TMBT merklich zurückgeht. Dadurch kann das Verfahren kostengünstiger betrieben werden, da weniger Katalysator benötigt wird.

Die erste Stufe des erfindungsgemäßen Verfahrens wird üblicherweise in CCl₄ als Lösungsmittel durchgeführt. Neben CCl₄ können jedoch weitere Lösungsmittel vorliegen. Geeignete weitere Lösungsmittel sind Halogenalkane wie Dichlormethan, Dichlorethan, Dibrommethan und Bromoform, halogenierte Aromaten wie Chlorbenzol, Kohlenwasserstoffe wie die isomeren Pentane, Hexane, Heptane, Octane sowie höhere Kohlenwasserstoffe mit mehr als 8 C-Atomen, Cyclohexan und Kohlenwasserstoffgemische wie Petrolether und Testbenzin. Die Gegenwart weiterer Lösungsmittel ist bevorzugt, falls nur mit einem geringen AlCl₃-Überschuss gearbeitet wird, also beispielsweise die Menge AlCl₃ gemäß einer Ausführungsform des erfindungsgemäßen Verfahrens nur 1 bis 1,5 Äquivalente beträgt. Bei dieser Verfahrensweise mit geringem AlCl₃-Überschuss kann durch die Anwesenheit von weiteren Lösungsmitteln das Ausfallen eines Komplexes des trichlormethylierten Aromaten (III) mit AlCl₃ oder anderen Lewis-Säuren, beispielsweise eines TMBT/AlCl₃-Komplexes, als Feststoff verhindert werden. Das Molverhältnis von weiterem Lösungsmittel zu CCl₄ kann zu Beginn der Umsetzung von 0,2 : 1 bis 10 : 1, bevorzugt von 0,5 : 1 bis 3 : 1 betragen.

Die erste Stufe des erfindungsgemäßen Verfahrens wird üblicher Weise bei einer Temperatur von 0 bis 120 °C, bevorzugt von 20 bis 60 °C durchgeführt. Dabei kann so verfahren werden, dass der Friedel-Crafts-Katalysator in CCl₄ bzw. in dem Gemisch aus CCl₄ und dem weiteren Lösungsmittel suspendiert vorgelegt wird und der substituierte Aromat (II) über einen gewissen Zeitraum, beispielsweise von 0,1 bis 10 Stunden, bevorzugt von 0,5 bis 5 Stunden zugegeben wird.

Wird die Friedel-Crafts-Alkylierung (erste Stufe) mit AlCl₃ als Katalysator durchgeführt, so wird der trichlormethylierte Aromat (III) als AlCl₃-Komplex erhalten. Es schließt sich an die Friedel-Crafts-Alkylierung die Hydrolyse dieses AlCl₃-Komplexes an. Diese Hydrolyse kann in üblicher Weise mit Eis oder einem Wasser/Eis-Gemisch beispielsweise bei einer Temperatur von 0 bis 10 °C durchgeführt werden. Die Hydrolyse kann beispielsweise diskontinuierlich durchgeführt werden, indem der Reaktionsaustrag der Friedel-Crafts-Alkylierung in einem diskontinuierlich betriebenen Rührapparat (Rührkessel) zu Eis gegeben wird.

In einer Ausführungsform der Erfindung wird die Hydrolyse des AlCl₃-Komplexes mit Wasser bei einer Temperatur von 10 bis 100 °C, vorzugsweise von 20 bis 100°C, besonders bevorzugt von 35 bis 80 °C durchgeführt. Es wurde überraschender Weise gefunden, dass die Hydrolyse des AlCl₃-Komplexes auch bei höheren Temperaturen von oberhalb 20 °C oder sogar oberhalb 35 °C durchgeführt werden kann, ohne dass eine Zersetzung des trichlormethylierten Aromaten (III) zur Carbonsäure stattfindet.

Die Durchführung der Hydrolyse bei höheren Temperaturen weist eine Reihe von Vorteilen auf. Die Handhabung von Eis im technischen Maßstab bedeutet einen hohen Aufwand. Auch die Durchführung der Hydrolyse bei Temperaturen unter 25°C bedeutet noch einen hohen Aufwand, da hier im allgemeinen die Kühlung mit Flusswasser nicht mehr ausreicht und Apparate mit entsprechend hoher Kühlleistung (Kühlaggregate, Solekühlung) erforderlich sind. Führt man die Hydrolyse bei zu tiefen Temperaturen durch, besteht die Gefahr, dass die Hydrolysereaktion einschläft, beim Erwärmen schlagartig anspringt und große Mengen HCl-Gas, das bei der Hydrolyse entsteht, freigesetzt werden, was im technischen Maßstab schwer zu handhaben ist und ein Sicherheitsproblem darstellt. Es ist daher wünschenswert, die Hydrolyse bei Temperaturen oberhalb von 20°C, bevorzug oberhalb von 35°C durchzuführen. Durch die bei den hohen Temperaturen höheren Rektionsgeschwindigkeiten der Hydrolyse sind entsprechend geringere Verweilzeiten möglich, so dass die Hydrolyse kontinuierlich in kostengünstigen kleinen, kontinuierlich betriebenen Apparaten, wie einem Mixer-Settler-Apparat, durchgeführt werden kann. Die kontinuierliche Verfahrensführung erlaubt auch eine bessere Kontrolle der Reaktion.

Bei der Hydrolyse werden eine organische und eine wässrige Phase erhalten. Die organische Phase enthält den trichlormethylierten Aromaten (III), gegebenenfalls bereits geringe Mengen des Arylcarbonsäurechlorids (I), nicht umgesetztes CCl₄ sowie gegebenenfalls das oder die weiteren Lösungsmittel.

Aus der organischen Phase kann der trichlormethylierte Aromat (III) in reiner Form als Zwischenprodukt isoliert werden, vorzugsweise durch Destillation.

In der zweiten Stufe des erfindungsgemäßen Verfahrens wird der trichlormethylierte Aromat (III) mit Wasser in Gegenwart eines Katalysators behandelt, wobei das Arylcarbonsäurechlorid (I) erhalten wird.

In der zweiten Stufe wird der trichlormethylierte Aromat (III) in reiner Form oder in Form einer Lösung des trichlormethylierten Aromaten (III) in CCl₄ und gegebenenfalls dem weiteren Lösungsmittel, wie sie bei der Hydrolyse des AlCl₃-Komplexes als organische Phase anfällt, eingesetzt.

Dadurch entfällt die aufwändige Isolierung des Zwischenproduktes (III). Überraschender Weise wurde gefunden, dass der Einsatz der organischen Phase aus der Hydrolyse des AlCl₃-Komplexes in der zweiten Stufe des erfindungsgemäßen Verfahrens keine Ausbeuteverluste mit sich bringt.

Diese Verfahrensweise bringt eine Reihe von Vorteilen mit sich. Isolierung des trichlormethylierten Aromaten (III) aus der Lösung in überschüssigem CCl₄ und gegebenenfalls einem weiteren Lösungsmittel bedeutet zusätzlichen verfahrenstechnischen Aufwand. Da die organische Phase nach Hydrolyse des AlCl₄-Komplexes mit überschüssigem Wasser wasserhaltig ist und dieses Wasser beim Abdestillieren gemeinsam mit CCl₄ als CCl₄/Wasser-Azeotrop mit übergeht, kann das abdestillierte CCl₄ nicht ohne weitere Trocknung erneut in die Friedel-Crafts-Alkylierung der ersten Stufe zurückgeführt werden. Durch den Einsatz der wasserhaltigen organischen Phase aus der Hydrolyse des AlCl₃-Komplexes in der zweiten Stufe des erfindungsgemäßen Verfahrens wird dieses Problem in eleganter Weise umgangen, da in der zweiten Stufe das in der organischen Phase enthaltene Wasser verbraucht wird. Die organische Phase wird auf diese Weise in der zweiten Stufe "chemisch" getrocknet. Anschließend kann völlig trockenes CCl₄ abdestilliert und in die erste Stufe zurückgeführt werden.

In der zweiten Stufe des erfindungsgemäßen Verfahrens wird der trichlormethylierte Aromat (III) mit Wasser behandelt, also hydrolysiert. Das Verhältnis Wasser zu trichlormethyliertem Aromat (III) beträgt dabei im Allgemeinen von 0,8 : 1 bis 1,2 : 1, bevorzugt von 0,9 : 1 bis 1,1 : 1, insbesondere ungefähr 1 : 1. Geeignete Katalysatoren sind Lewis-Säuren wie AlCl₃, FeCl₃ , AlBr₃, CoCl₃, LiCl, LiClO₄, SnCl₄, TiCl₄, ZrCl₄, SbCl₅, CoCl₂, BF₃, BCl₃ und ZnCl₂ und alle in George Olah, "Friedel Crafts and related reactions", Vol. 1, 201 und 284-290 (1963) beschriebenen Friedel-Crafts-Katalysatoren. Außerdem können auch Brönsted-Säuren als Katalysatoren eingesetzt werden. Geeignet sind beispielsweise Schwefelsäure, Phosphorsäure, Polyphosphorsäuren, Pyroschwefelsäure, Fluorschwefelsäure, Chlorsulfonsäure, Methansulfonsäure, p-Toluolsulfonsäure, Trifluoressigsäure und Trifluormethansäure. Bevorzugter Katalysator ist FeCl₃. Die Lewis-Säure liegt dabei im Allgemeinen in Mengen von 0,05 bis 5 mol-%, vorzugsweise 0,1 bis 3 mol-%, bezogen auf den trichlormethylierten Aromaten (III), vor. Wird FeCl₃ als Lewis-Säure eingesetzt, so kann diese auch als wässrige Lösung, beispielsweise als 30 gew.-%ige wässrige Lösung zugegeben werden. Die Umsetzung kann in Abwesenheit eines organischen Lösungsmittels durchgeführt werden. Es kann aber auch in CCl₄ bzw. in dem Gemisch aus CCl₄ und dem weiteren Lösungsmittel aus der ersten (Alkylierungs)Stufe des erfindungsgemäßen Verfahrens gearbeitet werden.

Die Umsetzungstemperatur bei der Hydrolyse (zweiten Stufe) beträgt im Allgemeinen von 20 bis 100°C, bevorzugt von 50 bis 75 °C. Im Falle der Herstellung von TMBC aus TMBT beträgt die Temperatur bei der Hydrolyse im Allgemeinen von 20 bis 100 °C, bevorzugt von 50 bis 75°C.

Aus der bei der Hydrolyse erhaltenen organischen Phase kann das Alkylbenzoylchlorid (I) durch Destillation in reiner Form gewonnen werden.

Durch das erfindungsgemäße Verfahren wird die bekannte vierstufige Synthese durch eine zweistufige Synthese ersetzt. Die Gesamtausbeute über beide Stufen einschließlich der Aufarbeitung kann dabei, bezogen auf eingesetzten substituierten Aromaten (II), > 80%, bevorzugt > 85% und sogar bis zu 90% betragen. Bei der Herstellung von TMBC wird beispielsweise eine Gesamtausbeute von 91%, verglichen mit 81,7% nach der bekannten vierstufigen Synthese, erreicht.

Die Erfindung wird durch die nachstehenden Beispiele näher erläutert.

### Beispiele

### Beispiel 1

### Herstellung von TMBT

183,3 g (1,375 mol; 1,1 Äquivalente) AlCl₃ werden in 1153,5 g (7,5 mol) CCl₄ bei 40°C suspendiert. Innerhalb von 83 min tropft man 150 g (1,25 mol) Mesitylen bei 40°C zu. Bereits nach den ersten Tropfen färbt sich der Ansatz dunkelrot und HCl-Entwicklung kann beobachtet werden. Nach Zugabe von 90% des Mesitylens fällt ein Feststoff aus. Die Suspension ist gut rührbar. Nach vollständiger Mesitylenzugabe wird noch 90 min bei 40°C nachgerührt. Die Reaktionsmischung wird auf 1500 g Eis/185 g konzentrierte HCl gegossen und hydrolysiert. Die Zugabe der Reaktionsmischung erfolgt so, dass die Temperatur nicht über 3°C steigt. Die wässrige Oberphase wird verworfen. Die organische Unterphase wird mit Magnesiumsulfat getrocknet (mit CCl₄ nachwaschen) und destilliert. Zur Destillation werden 1705,6 g organische Phase eingesetzt. Bei Normaldruck destillieren bei 76°C 1413,4 g CCl₄ ab. Anschließend werden bei ca. 1,6 mbar und ca. 98°C 268,2 g TMBT destilliert, das laut GC-Analyse eine Reinheit von 93,7 Flächen-% hat. Das TMBT enthält außerdem ca. 2,5 Flächen-% TMBC. Die isolierte Ausbeute an TMBT und TMBC beträgt insgesamt 87,6 %.

### Beispiel 2

### Hydrolyse von TMBT

268,2 g TMBT (93,7 Flächen-% TMBT = 1,053 mol + 2,5 Flächen-% TMBC) aus Beispiel 2 werden bei Raumtemperatur mit 0,3 g (0,0019 mol) FeCl₃ (wasserfrei) versetzt. Eine Rotfärbung kann beobachtet werden. Bei 60°C werden in 44 min 19,0 g (1,053 mol) VE-Wasser zugetropft, wobei Gasentwicklung einsetzt. Nach vollständiger Zugabe wird noch 105 min bei 60°C gerührt. Zur Vervollständigung des Umsatzes werden weitere 0,25 g Wasser zugegeben, bis laut GC-Analyse kein TMBT mehr vorhanden ist. Der Reaktionsaustrag wird ohne Kolonne bei 1,8 mbar und 72°C destilliert, wobei 196,4 g TMBC (96,0 Flächen-%) erhalten werden. Die Ausbeute beträgt 95,8 %. Zur weiteren Aufreinigung wird das TMBC noch mal über eine Kolonne destilliert, wobei 147,8 g TMBC mit einer Reinheit von 99,5 Flächen-% erhalten werden. Die Gesamtausbeute an isoliertem TMBC bezogen auf Mesitylen beträgt 83,9 %.

### Beispiel 3

### Herstellung von TMBT

216,7 g (1,625 mol; 1,3 Äquivalente) AlCl₃ werden in 1153,5 g (7,5 mol) CCl₄ bei 40°C suspendiert. Innerhalb von 35 min tropft man 150 g (1,25 mol) Mesitylen bei 40°C zu. Bereits nach den ersten Tropfen färbt sich der Ansatz dunkelrot und HCl-Entwicklung kann beobachtet werden. Nach vollständiger Mesitylenzugabe wird noch 90 min bei 40°C nachgerührt. Die Reaktionsmischung wird auf 2000 g Eis/300g konzentrierter HCl gegossen und hydrolysiert. Die Zugabe des Reaktionsaustrages erfolgt so, dass die Temperatur nicht über 3°C steigt. Die wässrige Oberphase wird verworfen. Die organische Unterphase wird mit Magnesiumsulfat getrocknet (mit CCl₄ nachwaschen) und destilliert. Bei Normaldruck destillieren bei 78°C 1331,4 g CCl₄ ab. Anschließend werden bei ca. 1,1 mbar und ca. 106°C 272,2 g TMBT destilliert, das laut GC-Analyse eine Reinheit von 91,9 Flächen-% hat. Das TMBT enthält außerdem ca. 4,7 Flächen-% TMBC. Die isolierte Ausbeute an TMBT und TMBC beträgt insgesamt 89,8 %.

### Beispiel 4

### Acidolyse von TMBT

258,5 g TMBT (91,9 Flächen-% TMBT = 1,0 mol; 4,7 Flächen-% TMBC) aus Beispiel 3 werden bei Raumtemperatur mit 0,2 g (0,0013 mol) FeCl₃ (wasserfrei) versetzt. Eine Rotfärbung kann beobachtet werden. Bei 70°C werden in ca. 2 Stunden 95,5 g (1,0 mol) Chlor essigsäure (CES) zugetropft, wobei Gasentwicklung einsetzt. Nach vollständiger Zugabe wird noch 60 min bei 70°C gerührt. Der Reaktionsaustrag wird destilliert. Bei 180 mbar und 58°C werden zunächst 105,5 g Chloressigsäurechlorid (CEC) abdestilliert. Diese Fraktion besteht zu 96,4 Flächen-% aus CEC und zu 3,5 Flächen-% aus TMBC. Das entspricht einer CEC-Ausbeute von 90 %. Anschließend werden bei 4,2 mbar und 86°C 196 g TMBC abdestilliert. Die isolierte TMBC Ausbeute beträgt 99,3 %. Die Gesamtausbeute an isoliertem TMBC bezogen auf Mesitylen beträgt 89,2 %.

### Beispiel 5

### Herstellung von TMBC ohne Isolierung von TMBT

250,0 g AlCl₃ (1,875 mol; 1,5 Äquivalente bezogen auf Mesitylen) werden in 288,4 g CCl₄ (1,875 mol; 1,5 Äquivalente bezogen auf Mesitylen) bei 24°C suspendiert. Innerhalb von 40 min tropft man 150 g (1,25 mol) Mesitylen bei 24-55°C zu. Bereits nach den ersten Tropfen färbt sich der Ansatz dunkelrot und HCl-Entwicklung kann beobachtet werden. Nach Zugabe des Mesitylens liegt eine gut rühr- und pumpbare, dünne Suspension vor. Es wird noch 90 min bei 43°C nachgerührt. Die Reaktionsmischung wird über einen Zeitraum von 20 min in eine Mischung aus 685 g VE-Wasser und 115 g konzentrierter HCl getropft. Die Hydrolyse wird bei Raumtemperatur gestartet und im weiteren Verlauf durch Kühlen auf unter 45°C gehalten. Die Hydrolysereaktion springt sofort an. Nach vollständiger Zugabe der Reaktionsmischung bilden sich zwei flüssige Phasen. Die untere braune organische Phase wird abgetrennt und ohne weitere Behandlung in einem Glaskolben erneut vorgelegt. Man erhitzt auf 50°C und gibt 1,0 g einer 30 gew.-%igen Lösung von FeCl₃ (0,00188 mol) in Wasser zu. Nach ca. 5 min setzt eine leichte Gasentwicklung ein. Nach Erhöhen der Temperatur auf 60°C werden in 33 min 15 g VE-Wasser zugetropft. Insgesamt werden noch weitere 4,5 g (0,25 mol) VE-Wasser zugetropft. Nach beendeter Zugabe rührt man 60 min nach. Das erhaltene Reaktionsgemisch wird nun destilliert. Zunächst destilliert man bei 120 mbar überschüssiges CCl₄ ab. Anschließend wird TMBC bei 0,25-0,4 mbar und einer Temperatur von 62-68°C abdestilliert. Insgesamt werden 208,1 g (1,14 mol) TMBC erhalten, was einer Ausbeute von 91,2% entspricht.

## Patentansprüche

1. Verfahren zur Herstellung von mit C₁-C₄-Alkyl 3-, 4- oder 5-fach oder mit Halogen ein- oder mehrfach substituierten Benzoylchloriden (I), bei dem in einer ersten Stufe ein mit C₁-C₄-Alkyl 3-, 4- oder 5-fach oder mit Halogen ein- oder mehrfach substituiertes Benzol (II) mit CCl₄ in Gegenwart eines von AlCl₃ und anschließender Hydrolyse des gebildeten AlCl₃-Komplexes zum entsprechenden, mit C₁-C₄-Alkyl 3-, 4- oder 5-fach oder mit Halogen ein- oder mehrfach substituierten trichlormethylierten Aromaten (III) umgesetzt wird,
und in einer zweiten Stufe das trichlormethylierte Benzol (III) mit Wasser in Gegenwart eines Katalysators zum Benzoylchlorid (I) hydrolysiert wird, wobei in der zweiten Stufe die bei der Hydrolyse des AlCl₃-Komplexes anfallende wasserhaltige organische Phase eingesetzt und nach der Hydrolyse wasserfreies CCl₄ abdestilliert wird.

2. Verfahren nach Anspruch 1, **dadurch gekennzeichnet, dass** Trimethylbenzoylchlorid der Formel (Ib) 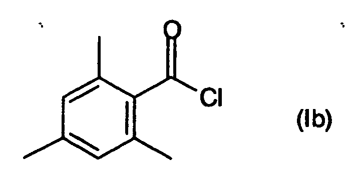 aus Mesitylen als substituiertem Benzol (II) hergestellt wird.

3. Verfahren nach Anspruch 1 oder 2, **dadurch gekennzeichnet, dass** das Molverhältnis CCl₄ zu substituiertem Aromat (II) von 1 : 1 bis 3,5 : 1 beträgt.

4. Verfahren nach einem der Ansprüche 1- 3, **dadurch gekennzeichnet, dass** 1 bis 1,5 Äquivalente AlCl₃ pro Äquivalent des substituierten Benzols (II) eingesetzt werden.

5. Verfahren nach Anspruch 3 oder 4, **dadurch gekennzeichnet, dass** der Komplex aus trichlormethyliertem Benzol (III) und AlCl₃ mit Wasser bei 20 bis 100 °C hydrolysiert wird.

6. Verfahren nach Anspruch 5, **dadurch gekennzeichnet, dass** die Hydrolyse des Komplexes aus trichlormethyliertem Aromaten (III) und AlCl₃ kontinuierlich durchgeführt wird.

7. Verfahren nach einem Ansprüche 1 bis 6, **dadurch gekennzeichnet, dass** in der zweiten Stufe FeCl₃ als Katalysator eingesetzt wird.

## Claims

1. A process for preparing tri-, tetra- or penta- C₁-C₄-alkyl- or mono- or poly-halogen-substituted benzoyl chlorides (I), by, in a first stage, reacting a tri-, tetra- or penta- C₁-C₄-alkyl- or mono- or poly-halogen-substituted benzene (II) with CCl₄ in the presence of AlCl₃ and subsequent hydrolysis of the AlCl₃ complex formed to give the corresponding tri-, tetra- or penta- C₁-C₄-alkyl- or mono- or poly-halogen-substituted trichloromethylated aromatic (III), and, in a second stage, hydrolyzing the trichloromethylated benzene (III) with water in the presence of a catalyst to obtain the benzoyl by, in the second stage, using the aqueous organic phase obtained in the hydrolysis of the AlCl₃ complex and, after the hydrolysis, distilling off anhydrous CCl₄

2. The process according to Claim 1, wherein trimethylbenzoyl chloride of the formula (Ib) 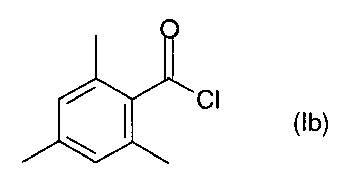 is prepared from mesitylene as the substituted benzene (II).

3. The process according Claim 1 or 2, wherein the molar ratio of CCl₄ to substituted aromatic (II) is from 1:1 to 3.5:1.

4. The process according to any of Claims 1 to 3, wherein from 1 to 1.5 equivalents of AlCl₃ per equivalent of the substituted benzene (II) are used.

5. The process according to Claim 3 or 4, wherein the complex of trichloromethylated benzene (III) and AlCl₃ is hydrolyzed with water at from 20 to 100°C.

6. The process according to Claim 5, wherein the hydrolysis of the complex of trichloromethylated aromatic (III) and AlCl₃ is carried out continuously.

7. The process according to any of Claim 6, wherein the catalyst used in the second stage is FeCl₃.

## Revendications

1. Procédé de fabrication de chlorures de benzoyle (I) substitués 3, 4 ou 5 fois avec un alkyle en C₁ à C₄ ou une ou plusieurs fois avec un halogène, selon lequel lors d'une première étape, un benzène (II) substitué 3, 4 ou 5 fois avec un alkyle en C₁ à C₄ ou une ou plusieurs fois avec un halogène est transformé avec CCl₄, en présence d'AlCl₃ et avec hydrolyse ultérieure du complexe d'AlCl₃ formé, en composé aromatique trichlorométhylé (III) correspondant, substitué 3, 4 ou 5 fois avec un alkyle en C₁ à C₄ ou une ou plusieurs fois avec un halogène,
et lors d'une seconde étape, le benzène trichlorométhylé (III) est hydrolysé avec de l'eau en présence d'un catalyseur en chlorure de benzoyle (I), la phase organique contenant de l'eau qui se forme lors de l'hydrolyse du complexe d'AlCl₃ étant utilisée lors de la seconde étape et du CCl₄ anhydre étant éliminé par distillation après l'hydrolyse.

2. Procédé selon la revendication 1, **caractérisé en ce que** le chlorure de triméthylbenzoyle de formule (Ib) 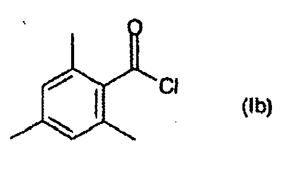 est fabriqué à partir de mésitylène en tant que benzène substitué (II).

3. Procédé selon la revendication 1 ou 2, **caractérisé en ce que** le rapport molaire entre CCl₄ et le composé aromatique substitué (II) est de 1:1 à 3,5:1.

4. Procédé selon l'une quelconque des revendications 1 à 3, **caractérisé en ce qu'**1 à 1,5 équivalents d'AlCl₃ sont utilisés par équivalent du benzène substitué (II).

5. Procédé selon la revendication 3 ou 4, **caractérisé en ce que** le complexe de benzène trichlorométhylé (III) et d'AlCl₃ est hydrolysé avec de l'eau à 20 à 100°C.

6. Procédé selon la revendication 5, **caractérisé en ce que** l'hydrolyse du complexe du composé aromatique trichlorométhylé (III) et d'AlCl₃ est réalisée en continu.

7. Procédé selon l'une quelconque des revendications 1 à 6, **caractérisé en ce que** du FeCl₃ est utilisé en tant que catalyseur lors de la seconde étape.
